# EUROPEAN PATENT APPLICATION

(11) **EP 1 113 074 A1**
(43) Date of publication of application: **04.07.2001**
(21) Application number: 99204612.8
(22) Date of filing: 30.12.1999
(51) Int. Cl.: C12N 15/31, C07K 14/285, C12N 1/21, C07K 16/12, A61K 39/102, C12Q 1/68, G01N 33/569

(54) **Actinobacillus pleuropneumoniae outer membrane protein and its use**

(71) Applicant: UNIVERSITEIT GENT, 9000 Gent (BE)
(72) Inventor: Haesebrouck, Freddy, 9820 Merelbeke (BE); Ducatelle, Richard, 9700 Oudenaarde (BE); Chiers, Koen, 8510 Marke (BE); Van Overbeke, Ingrid, 9880 Aalter (BE)
(74) Representative: De Clercq, Ann

(57) **Abstract**

The present invention relates to a new *Actinobacillus pleuropneumoniae* outer membrane protein having a molecular weight of about 55 kDa and having an N-terminal sequence as shown in SEQ ID NO 1. The invention also relates to nucleic acids encoding said protein and the use of both types of molecules for the treatment and prevention of pleuropneumonia infections in pigs.

### Annex to the application documents-subsequently filed sequences listing

SEQUENCE LISTING

<110> Universiteit Gent

<120> New actinobacillus pleuropneumoniae outer membrane
      protein and its uses

<130> EP.99.UG.7.ACT

<140> EP 99204612.8
<141> 1999-12-30

<160> 1

<170> PatentIn Ver. 2.1

<210> 1
<211> 26
<212> PRT
<213> Actinobacillus pleuropneumoniae

<220>
<221> MOD_RES
<222> (19)
<223> probably Gln

<220>
<221> MOD_RES
<222> (21)
<223> Gln or Ala

<220>
<221> MOD_RES
<222> (25)
<223> Gly or Asp

<400> 1
Gly Lys Asp Leu Asn Val Phe Asp Lys Asn Tyr Gly Leu Leu Ile Asn
  1               5                  10                  15

Gly Lys Gln Thr Xaa Phe Arg Ser Xaa Asp
             20                  25

## Description

### Field of the invention

The present invention relates to the field of molecular biology and bacteriology. More particularly, the present invention relates to the treatment, prevention and detection of *Actinobacillus pleuropneumoniae* infections in pigs. The present invention relates more particularly relates to the characterisation of a new outer membrane protein of *Actinobacillus pleuropneumoniae* as well nucleic acids encoding the same and the use of both types of molecules, particularly for treatment and prevention of pleuropneumonia in pigs.

### Background of the invention

*Actinobacillus pleuropneumoniae* is a Gram negative bacterium causing contagious pleuropneumonia in pigs. Based on NAD requirements, *A. pleuropneumoniae* can be divided into biotype 1 strains, which are NAD-dependent, and biotype 2 strains, which are NAD-independent. So far 14 serotypes have been described. Serotypes 1 and 5 are subdivided into 1a, 1b and 5a, 5b (17, 19).

In previous studies, it was shown that *A. pleuropneumoniae* adheres to the epithelium of the alveoli or the cilia of the terminal bronchioli of experimentally infected pigs (9). It has also been shown that *A. pleuropneumoniae* adheres to porcine tracheal rings maintained in culture (1), to porcine frozen tracheal and lung sections (15), to erythrocytes from various animal species (16), to tonsillar epithelial cells (4), to swine buccal epithelial cells (13) and to type III swine-lung collagen (11).

Adherence to host tissue is generally regarded as an important prerequisite for colonization and virulence manifestation of bacteria (20). Adherence enables colonization to occur and allows the bacterium to exert its pathogenic effects.

Several surface structures have been described in the family of *Pasteurellaceae* that are involved in adhesion, including the capsule (5), fimbriae (24), lipopolysaccharides (1) and outer membrane proteins (5). Concerning *A. pleuropneumoniae*, only few adhesion factors have been described. Lipopolysaccharides seem to be involved in the in vitro adhesion to porcine tracheal rings (1) and mucus (3, 2). Recently, it was demonstrated that the capsule is not responsible for adherence to tracheal frozen sections (14). Fimbriae have been demonstrated on *A. pleuropneumoniae* (10) but their role in adherence needs to be elucidated.

Since in vivo studies indicated that adherence of *A. pleuropneumoniae* to alveolar epithelial cells constitutes an important initial step in pathogenesis (9, 2), the association of the bacterium with these host cells was further characterized in the present studies, using an in vitro adhesion model.

### Summary of the invention

The present invention provides a purifed immunogenic *Actinobacillus pleuropneumoniae* outer membrane protein having an N-terminal amino acid sequence as shown in SEQ ID NO 1 and having a molecular weight of about 55 kD or a fragment thereof .

Said fragments according to the present invention are preferable immunogenic fragments or fragments being specifically recognized by sera from *Actinobacillus pleuropneumoniae* infected individuals, and/or those fragments being specifically recognized by the cellular immune response from *Actinobacillus pleuropneumoniae* contacted animals. Also preferred fragments according to the present invention are those being able to elicit an *Actinobacillus pleuropneumoniae* specific immune response when used for vaccination of individuals prone to pleuropneumonia. Said fragments are preferably also fragments having a unique sequence meaning that said fragment does not share full amino acid sequence homology (identity) with sequences of known proteins.

Said protein according to the present invention is further characterized in that it is particularly expressed under NAD restricted conditions as set out in the Examples section.

The present invention further relates to a polynucleic acid comprising a sequence of at least 10 contiguous nucleotides selected from:
(a) a polynucleic acid sequence which codes for a polypeptide as defined above,
(b) a polynucleic acid sequence which is degenerate as a result of the genetic code to the polynucleic acid sequence as defined in (a), and which still encodes a polypeptide as defined above, or
(c) a polynucleic acid sequence which hybridizes to any of the polynucleic acids as defined in (a) or (b).

Preferably said polynucleic acid sequence hybridizes to any of the polynucleic acids as defined in (a) or (b) under stringent hybridisation conditions well known to the man skilled in the art.

The present invention further relates to an oligonucleotide probe comprising a fragment of a polynucleic acid as defined above, with said probe being able to act as a specific hybridization probe for detecting the presence of *Actinobacillus pleuropneumoniae* polynucleic acids in a sample.

The present invention further relates to an oligonucleotide primer comprising a fragment of a polynucleic acid as defined above, with said primer being able to initiate specific amplification of *Actinobacillus pleuropneumoniae* polynucleic acids in a sample.

The present invention also relates to an antibody, more particularly a monoclonal antibody, characterized in that it specifically recognizes an *Actinobacillus pleuropneumoniae* polypeptide as defined above, or an immunogenic fragment thereof or any other fragment as defined above.

The present invention moreover relates to a method for (in vitro) detection of *Actinobacillus pleuropneumoniae* comprising the steps of contacting a sample taken from said individual with a polypeptide or fragment thereof as defined above under conditions allowing the formation of an immunological complex, and subsequently detecting the complexes formed.

The present invention further relates to a method for (in vitro) detection of *Actinobacillus pleuropneumoniae* comprising the steps of contacting a sample taken from an individual with an oligonucleotide probe as defined above, under conditions allowing the formation of a hybridization complex, with said polynucleic acids of said sample being possibly amplified prior to hybridization, and subsequently detecting the complexes formed.

The present invention further relates to a method for (in vitro) detection of *Actinobacillus pleuropneumoniae* comprising the steps of contacting a sample taken from an individual with an oligonucleotide primer as defined above, under conditions allowing the specific amplification of polynucleic acids, and sequencing the amplified polynucleic acids or detecting the amplified polynucleic acids with suitable nucleotide probes.

The present invention further relates to a method for (in vitro) diagnosis of *Actinobacillus pleuropneumoniae* infection in an individual comprising the steps of contacting a sample taken from said individual, with an antibody as defined above, under conditions allowing the formation of an immunological complex, and subsequently detecting the complexes formed.

The present invention further relates to a method for detecting and/or quantifying the cellular immune response of an individual against a polypeptide as defined above, said method comprising:
- measuring a delayed type hypersensitivity reaction upon subcutaneous injection of said polypeptide in said individual, or
- measuring the stimulation of periferal blood lymphocytes isolated from the individual to be tested, upon addition of said polypeptide to said lymphocytes under conditions allowing the immune recognition of said polypeptide.

The present invention further relates to a method for detecting individuals having been in contact with *Actinobacillus pleuropneumoniae* comprising:
- contacting a polypeptide as defined above with the cellular immune system of said individual, either in vitro or in vivo, and,
- detecting and/or quantifying the cellular immune response raised against said polypeptides by means of a method as defined above.

The present invention further relates to a recombinant *Actinobacillus pleuropneumoniae* strain in which the gene(s) encoding at least one of the polypeptides as defined above has (have) been deleted or inactivated.

The present invention further relates to a vaccine composition comprising as an active component a polypeptide as defined above or a fragment thereof, or a polynucleic acid as defined above, or a recombinant *Actinobacillus pleuropneumoniae* strain as defined above, said active component being combined with a pharmaceutically acceptable carrier.

The present invention further relates to a combined method for vaccination against and detection of pleuropneumonia, said method comprising:
- vaccinating an individual liable to become infected with pleuropneumonia with a recombinant *Actinobacillus pleuropneumoniae* strain as defined above, and,
- detecting in an individual liable to be infected with pleuropneumoniae an immune response against a polypeptide as defined above, by means of a method as defined above, wherein said combined method is characterized in that it enables the differentiation between vaccinated and field-infected individuals.

The present invention further relates to a DNA construct comprising a polynucleic acid as defined above, or a part thereof and wherein the coding sequence of said polynucleic acid is operably linked to a control sequence enabling the expression of the coding sequence by a specific host. Suitable hosts are well known in the art.

The present invention also relates to a host cell transformed with a DNA construct as defined above.

Said host cell being preferably a prokaryotic organism, and more preferably E. coli, a Salmonella species or a lactic acid bacterium, or with said host cell being a lower eukaryotic cell (like yeast) or a higher eukaryotic cell.

The present invention also relates to a recombinant polypeptide encoded by a polynucleic acid as defined above or part thereof, said recombinant polypeptide being produced by:
- transforming a DNA construct as defined above into a suitable host cell,
- culturing said transformed cellular host under conditions, which allow the expression and possibly secretion of the encoded polypeptide, and
- recovering the expressed polypeptide from the culture.

The present invention also relates to a kit for the detection of *Actinobacillus pleuropneumoniae* comprising a polypeptide or peptide as defined above, or a recombinant peptide as defined above as defined above, preferentially in combination with other polypeptides or peptides from *Actinobacillus pleuropneumoniae*, with said (poly)peptides being preferably immobilized on a solid substrate.

The present invention also relates to a kit for the detection of *Actinobacillus pleuropneumoniae* comprising an antibody as defined above, with said antibody being preferably bound to a solid support.

The present invention also relates to a kit for the detection of *Actinobacillus pleuropneumoniae* comprising:
- a primer as defined above, and/or
- an oligonucleotide probe as defined above, with said probe being preferentially immobilized on a solid substrate.

The present invention also relates to a kit for combined vaccination against and detection of pleuropneumonia, with said kit comprising at least the following components:
- a vaccine composition comprising as an active principle a recombinant *Actinobacillus pleuropneumoniae* strain as defined above, and,
- a polypeptide or peptide as defined above, or a recombinant polypeptide as defined above,
said kit being further characterized by the fact that its components enable the differentiation between vaccinated and field-infected individuals.

The present invention also relates to the combined use of:
- a recombinant *Actinobacillus pleuropneumoniae* vaccine strain as defined above for use in vaccination, and,
- a polypeptide or peptide as defined above or a recombinant polypeptide as defined above for use in a diagnostic method.

The present invention also relates to a polypeptide or fragment thereof as defined above or a recombinant protein as defined above, or an antibody as defined above, or a polynucleic acid as defined above, for use as a medicament.

The present invention also relates to a polypeptide or a fragment thereof according to claim 1 or a recombinant polypeptide as defined above, or an antibody as defined above, or a polynucleic acid as defined above, for the manufacture of a medicament, more particularly for the preparation of a vaccine or for the preparation of a diagnostic composition.

The present invention also relates to a method for producing an immunogenic *Actinobacillus pleuropneumoniae* outer membrane protein as defined above comprising growing a host cell as defined above under conditions whereby the protein encoded by said DNA construct is expressed, thereby producing said outer membrane protein as defined above.

### Detailed description of the invention

The practice of the present invention will employ, unless otherwise indicated, conventional techniques of molecular biology, microbiology, virology, recombinant DNA technology, and immunology, which are within the skill of the art. Such techniques are explained fully in the literature.

All publications cited herein are hereby incorporated by reference in their entirety. In describing the present invention, the following terms will be employed, and are intended to be defined as indicated below.

The terms "outer membrane protein" of the invention and "polypeptide" of the invention are equivalent and interchangeable and define proteins within the scope of claim 1. These terms also capture proteins substantially homologous and functionally equivalent to native proteins falling under claim 1. Thus, the term encompasses modifications, such as deletions, additions and substitutions (generally conservative in nature), to the native sequences, as long as immunological activity (as defined below) is not destroyed. Such modifications of the primary amino acid sequence may result in antigens which have enhanced activity as compared to the native sequence. These modifications may be deliberate, as through site-directed mutagenesis, or may be accidental, such as through mutations of hosts which produce the protein. All of these modifications are included, so long as immunogenic activity is retained.

Two nucleotide or amino acid sequences are "substantially homologous" when at least about 65% (preferably at least about 80% to 90%, and most preferably at least about 95%) of the nucleotides or amino acids match over a defined length of the molecule. As used herein, substantially homologous also refers to sequences showing identity to the specified DNA or polypeptide sequence. DNA sequences that are substantially homologous can be identified in a Southern hybridization experiment under, for example, stringent conditions, as defined for that particular system.

The term "functionally equivalent" intends that the amino acid sequence of the subject protein is one that will elicit an immunological response, as defined below, equivalent to or better than, the immunological response elicited by a native *A. pleuropneumoniae* outer membrane protein of the invention.

An "antigen" refers to a molecule containing one or more epitopes that will stimulate a host's immune system to make a humoral and/or cellular antigen-specific response. The term is also used interchangeably with "immunogen."

By "subunit antigen" is meant an antigen entity separate and discrete from a whole bacterium (live or killed). Thus, an antigen contained in a cell free extract would constitute a "subunit antigen" as would a substantially purified antigen.

A "hapten" is a molecule containing one or more epitopes that does not stimulate a host's immune system to make a humoral or cellular response unless linked to a carrier.

The term "epitope" refers to the site on an antigen or hapten to which a specific antibody molecule binds. The term is also used interchangeably with "antigenic determinant" or "antigenic determinant site."

An "immunological response" to an antigen or vaccine is the development in the host of a cellular and/or antibody-mediated immune response to the composition or vaccine of interest. Usually, such a response includes but is not limited to one or more of the following effects; the production of antibodies, B cells, helper T cells, suppressor T cells, and/or cytotoxic T cells, directed specifically to an antigen or antigens included in the composition or vaccine of interest.

The terms "immunogenic polypeptide" and "immunogenic amino acid sequence" refer to a polypeptide or amino acid sequence, respectively, which elicit antibodies that neutralize bacterial infectivity, and/or mediate antibody-complement or antibody dependent cell cytotoxicity to provide protection of an immunized host. An "immunogenic polypeptide" as used herein, includes the full length (or near full length) sequence of an *A*. *pleuropneumoniae* proteins of the invention, or an immunogenic fragment thereof. By "immunogenic fragment" is meant a fragment of an *A*. *pleuropneumoniae* protein of the invention which includes one or more epitopes and thus elicits antibodies that neutralize bacterial infectivity, and/or mediate antibody-complement or antibody dependent cell cytotoxicity to provide protection of an immunized host. Such fragments will usually be at least about 5 amino acids in length, and preferably at least about 10 to 15 amino acids in length. There is no critical upper limit to the length of the fragment, which could comprise nearly the full length of the protein sequence, or even a fusion protein comprising fragments of two or more of the *A*. *pleuropneumoniae* subunit antigens.

The terms "polypeptide" and "protein" are used interchangeably and refer to any polymer of amino acids (dipeptide or greater) linked through peptide bonds. Thus, the terms "polypeptide" and "protein" include oligopeptides, protein fragments, analogs, muteins, fusion proteins and the like.

By "purified protein" is meant a protein separate and discrete from a whole organism (live or killed) with which the protein is normally associated in nature. Thus, a protein produced synthetically or recombinantly would constitute a purified protein. The protein of the invention present in an extract is not a purified protein.

"Recombinant" polypeptides refer to polypeptides produced by recombinant DNA techniques; i.e., produced from cells transformed by an exogenous DNA construct encoding the desired polypeptide. "Synthetic" polypeptides are those prepared by chemical synthesis.

A "replicon" is any genetic element (e.g., plasmid, chromosome, virus) that functions as an autonomous unit of DNA replication in vivo; i.e., capable of replication under its own control.

A "vector" is a replicon, such as a plasmid, phage, or cosmid, to which another DNA segment may be attached so as to bring about the replication of the attached segment. The term "comprising" within the context of the present invention is to be understood as containing at least an item or step but possibly also containing more than that item or step. Comprising thus constitutes open language.

Central to the present invention is the discovery of a new family of *A. pleuropneumoniae* outer membrane proteins.

This protein, analogs thereof and/or immunogenic fragments derived from the protein, are provided in subunit vaccine compositions and thus problems inherent in prior vaccine compositions, such as localized and systemic side reactions, as well as the inability to protect against chronic disease, may be avoided. The vaccine compositions can be used to treat or prevent *A. pleuropneumoniae-induced* respiratory diseases in swine such as porcine pleuropneumonia. The antigens or antibodies thereto can also be used as diagnostic reagents to detect the presence of an *A*. *pleuropneumoniae* infection in a subject. Similarly, the genes from the various serotypes encoding the proteins of the invention can be cloned and used to design probes for the detection of *A. pleuropneumoniae* in tissue samples as well as for the detection of homologous genes in other bacterial strains. The subunit antigens can be conveniently produced by recombinant techniques, as described herein. The proteins of interest are produced in high amounts in transformants, do not require extensive purification or processing, and do not cause lesions at the injection site or other ill effects.

In order to identify genes encoding the subject proteins, recombinant techniques can be employed. These techniques are well known in the art and include DNA library screening or PCR cloning all well known in the art.

For example a DNA library can be prepared which consists of genomic DNA from an *A. pleuropneumoniae* serotype. The resulting clones can be used to transform an appropriate host, such as E. coli. Individual colonies can then be screened in an immunoblot assay, using polyclonal serum or monoclonal antibodies, to the desired antigen.

More specifically, after preparation of a DNA library, DNA fragments of a desired length are isolated by, e.g., sucrose density gradient centrifugation. These fragments are then ligated into any suitable expression vector or replicon and thereafter the corresponding host cell is transformed with the constructed vector or replicon. Transformed cells are plated in suitable medium. A replica plate must also be prepared because subsequent procedures kill these colonies. The colonies are then lysed in one of a number of ways, e.g., by exposure to chloroform vapor. This releases the antigen from the positive colonies. The lysed colonies are incubated with the appropriate unlabelled antibody and developed using an appropriate anti-immunoglobulin conjugate and substrate. Positively reacting colonies thus detected can be recovered from the replica plate and subcultured. Physical mapping, construction of deletion derivatives and nucleotide sequencing can be used to characterize the encoding gene.

An alternative method to identify genes encoding the proteins of the present invention, once the genomic DNA library is constructed as described above, is to prepare oligonucleotides to probe the library and to use these probes to isolate the gene encoding the desired protein. The basic strategies for preparing oligonucleotide probes, as well as screening libraries using nucleic acid hybridization, are well known to those of ordinary skill in the art. The particular nucleotide sequences selected are chosen so as to correspond to the codons encoding a known amino acid sequence from the desired protein. Since the genetic code is degenerate, it will often be necessary to synthesize several oligonucleotides to cover all, or a reasonable number of, the possible nucleotide sequences which encode a particular region of the protein. Thus, it is generally preferred in selecting a region upon which to base the probes, that the region not contain amino acids whose codons are highly degenerate. In certain circumstances, one of skill in the art may find it desirable to prepare probes that are fairly long, and/or encompass regions of the amino acid sequence which would have a high degree of redundancy in corresponding nucleic acid sequences, particularly if this lengthy and/or redundant region is highly characteristic of the protein of interest. It may also be desirable to use two probes (or sets of probes), each to different regions of the gene, in a single hybridization experiment. Automated oligonucleotide synthesis has made the preparation of large families of probes relatively straightforward. While the exact length of the probe employed is not critical, generally it is recognized in the art that probes from about 14 to about 20 base pairs are usually effective. Longer probes of about 25 to about 60 base pairs are also used. The selected oligonucleotide probes are labeled with a marker, such as a radionucleotide or biotin using standard procedures. The labeled set of probes is then used in the screening step, which consists of allowing the single-stranded probe to hybridize to isolated ssDNA from the library, according to standard techniques. Either stringent or permissive hybridization conditions could be appropriate, depending upon several factors, such as the length of the probe and whether the probe is derived from the same species as the library, or an evolutionarily close or distant species. The selection of the appropriate conditions is within the skill of the art. The basic requirement is that hybridization conditions be of sufficient stringency so that selective hybridization occurs; i.e., hybridization is due to a sufficient degree of nucleic acid homology (e.g., at least about 75%), as opposed to nonspecific binding. Once a clone from the screened library has been identified by positive hybridization, it can be confirmed by restriction enzyme analysis and DNA sequencing that the particular library insert contains a gene for the desired protein.

Alternatively, DNA sequences encoding the proteins of interest can be prepared synthetically rather than cloned. The DNA sequence can be designed with the appropriate codons for the particular amino acid sequence. In general, one will select preferred codons for the intended host if the sequence will be used for expression. The complete sequence is assembled from overlapping oligonucleotides prepared by standard methods and assembled into a complete coding sequence.

Once coding sequences for the desired proteins have been prepared or isolated, they can be cloned into any suitable vector or replicon. Numerous cloning vectors are known to those of skill in the art, and the selection of an appropriate cloning vector is a matter of choice.

The gene can be placed under the control of a promoter, ribosome binding site (for bacterial expression) and, optionally, an operator (collectively referred to herein as "control" elements), so that the DNA sequence encoding the desired protein is transcribed into RNA in the host cell transformed by a vector containing this expression construction. The coding sequence may or may not contain a signal peptide or leader sequence. Leader sequences can be removed by the host in post-translational processing.

In addition to control sequences, it may be desirable to add regulatory sequences which allow for regulation of the expression of the protein sequences relative to the growth of the host cell. Regulatory sequences are known to those of skill in the art, and examples include those which cause the expression of a gene to be turned on or off in response to a chemical or physical stimulus, including the presence of a regulatory compound. Other types of regulatory elements may also be present in the vector, for example, enhancer sequences.

An expression vector is constructed so that the particular coding sequence is located in the vector with the appropriate regulatory sequences, the positioning and orientation of the coding sequence with respect to the control sequences being such that the coding sequence is transcribed under the "control" of the control sequences (i.e., RNA polymerase which binds to the DNA molecule at the control sequences transcribes the coding sequence). Modification of the sequences encoding the particular antigen of interest may be desirable to achieve this end. For example, in some cases it may be necessary to modify the sequence so that it may be attached to the control sequences with the appropriate orientation; i.e., to maintain the reading frame. The control sequences and other regulatory sequences may be ligated to the coding sequence prior to insertion into a vector, such as the cloning vectors described above. Alternatively, the coding sequence can be cloned directly into an expression vector which already contains the control sequences and an appropriate restriction site.

In some cases, it may be desirable to add sequences which cause the secretion of the polypeptide from the host organism, with subsequent cleavage of the secretory signal. It may also be desirable to produce mutants or analogs of the antigens of interest. Mutants or analogs may be prepared by the deletion of a portion of the sequence encoding the protein, by insertion of a sequence, and/or by substitution of one or more nucleotides within the sequence. Techniques for modifying nucleotide sequences, such as site-directed mutagenesis, are well known to those skilled in the art.

Depending on the expression system and host selected, the proteins of the present invention are produced by growing host cells transformed by an expression vector described above under conditions whereby the protein of interest is expressed. The protein is then isolated from the host cells and purified. If the expression system secretes the protein into growth media, the protein can be purified directly from the media. If the protein is not secreted, it is isolated from cell lysates. The selection of the appropriate growth conditions and recovery methods are within the skill of the art. The proteins of the present invention may also be produced by chemical synthesis such as solid phase peptide synthesis, using known amino acid sequences or amino acid sequences derived from the DNA sequence of the genes of interest. Such methods are known to those skilled in the art. Chemical synthesis of peptides may be preferable if a small fragment of the antigen in question is capable of raising an immunological response in the subject of interest.

The proteins of the present invention or their fragments can be used to produce antibodies, both polyclonal and monoclonal. If polyclonal antibodies are desired, a selected mammal, (e.g., mouse, rabbit, goat, horse, pig etc.) is immunized with an antigen of the present invention, or its fragment, or a mutated antigen. Serum from the immunized animal is collected and treated according to known procedures. If serum containing polyclonal antibodies is used, the polyclonal antibodies can be purified by immunoaffinity chromatography, using known procedures.

Monoclonal antibodies to the proteins of the present invention, and to the fragments thereof, can also be readily produced by one skilled in the art. The general methodology for making monoclonal antibodies by using hybridoma technology is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against the antigen of interest, or fragment thereof, can be screened for various properties; i.e., for isotype, epitope, affinity, etc. Monoclonal antibodies are useful in purification, using immunoaffinity techniques, of the individual antigens which they are directed against.

Animals can be immunized with the compositions of the present invention by administration of the protein of interest, or a fragment thereof, or an analog thereof. If the fragment or analog of the protein is used, it will include the amino acid sequence of an epitope which interacts with the immune system to immunize the animal to that and structurally similar epitopes.

If synthetic or recombinant proteins are employed, the subunit antigen can be a single polypeptide encoding one or several epitopes from one or more proteins of the invention or two or more discrete polypeptides encoding different epitopes. The subunit antigen, even though carrying epitopes derived from a lipoprotein, does not require the presence of the lipid moiety. However, if the lipid is present, it need not be a lipid commonly associated with the lipoprotein, so long as the appropriate immunologic response is elicited.

Prior to immunization, it may be desirable to increase the immunogenicity of the particular protein, or an analog of the protein, or particularly fragments of the protein. This can be accomplished in any one of several ways known to those of skill in the art. For example, the antigenic peptide may be administered linked to a carrier. Suitable carriers are typically large, slowly metabolized macromolecules such as: proteins; polysaccharides, such as sepharose, agarose, cellulose, cellulose beads and the like; polymeric amino acids such as polyglutamic acid, polylysine, and the like; amino acid copolymers; and inactive virus particles.

Especially useful protein substrates are serum albumins, keyhole limpet hemocyanin, immunoglobulin molecules, thyroglobulin, ovalbumin, and other proteins well known to those skilled in the art.

The protein substrates may be used in their native form or their functional group content may be modified by, for example, succinylation of lysine residues or reaction with Cys-thiolactone. A sulfhydryl group may also be incorporated into the carrier (or antigen) by, for example, reaction of amino functions with 2-iminothiolane or the N-hydroxysuccinimide ester of 3-(4-dithiopyridyl propionate. Suitable carriers may also be modified to incorporate spacer arms (such as hexamethylene diamine or other bifunctional molecules of similar size) for attachment of peptides.

Still other suitable carriers include cells, such as lymphocytes, since presentation in this form mimics the natural mode of presentation in the subject, which gives rise to the immunized state. Alternatively, the proteins of the present invention may be coupled to erythrocytes, preferably the subject's own erythrocytes. Methods of coupling peptides to proteins or cells are known to those of skill in the art. The novel proteins of the instant invention can also be administered via a carrier virus which expresses the same. Carrier viruses which will find use with the instant invention include but are not limited to the vaccinia and other pox viruses, adenovirus, and herpes virus.

It is also possible to immunize a subject with a protein of the present invention, or a protective fragment thereof, or an analog thereof, which is administered alone, or mixed with a pharmaceutically acceptable vehicle or excipient. Typically, vaccines are prepared as injectables, either as liquid solutions or suspensions; solid forms suitable for solution in, or suspension in, liquid vehicles prior to injection may also be prepared. The preparation may also be emulsified or the active ingredient encapsulated in liposome vehicles. The active immunogenic ingredient is often mixed with vehicles containing excipients which are pharmaceutically acceptable and compatible with the active ingredient. Suitable vehicles are, for example, water, saline, dextrose, glycerol, ethanol, or the like, and combinations thereof. In addition, if desired, the vehicle may contain minor amounts of auxiliary substances such as wetting or emulsifying agents, pH buffering agents, or adjuvants which enhance the effectiveness of the vaccine. Adjuvants may include for example, muramyl dipeptides, avridine, aluminum hydroxide, oils, saponins and other substances known in the art. Actual methods of preparing such dosage forms are known, or will be apparent, to those skilled in the art.

Additional vaccine formulations which are suitable for other modes of administration include suppositories and, in some cases, aerosol, intranasal, oral formulations, and sustained release formulations. For suppositories, the vehicle composition will include traditional binders and carriers, such as, polyalkaline glycols, or triglycerides. Such suppositories may be formed from mixtures containing the active ingredient in the range of about 0.5% to about 10% (w/w), preferably about 1% to about 2%. Oral vehicles include such normally employed excipients as, for example, pharmaceutical grades of mannitol, lactose, starch, magnesium, stearate, sodium saccharin cellulose, magnesium carbonate, and the like. These oral vaccine compositions may be taken in the form of solutions, suspensions, tablets, pills, capsules, sustained release formulations, or powders, and contain from about 10% to about 95% of the active ingredient, preferably about 25% to about 70%.

Intranasal formulations will usually include vehicles that neither cause irritation to the nasal mucosa nor significantly disturb ciliary function. Diluents such as water, aqueous saline or other known substances can be employed with the subject invention. The nasal formulations may also contain preservatives such as, but not limited to, chlorobutanol and benzalkonium chloride. A surfactant may be present to enhance absorption of the subject proteins by the nasal mucosa.

Controlled or sustained release formulations are made by incorporating the protein into carriers or vehicles such as liposomes, nonresorbable impermeable polymers such as ethylenevinyl acetate copolymers and Hytrel.RTM. copolymers, swellable polymers such as hydrogels, or resorbable polymers such as collagen and certain polyacids or polyesters such as those used to make resorbable sutures. The proteins can also be delivered using implanted mini-pumps, well known in the art.

Furthermore, the proteins (or complexes thereof) may be formulated into vaccine compositions in either neutral or salt forms. Pharmaceutically acceptable salts include the acid addition salts (formed with the free amino groups of the active polypeptides) and which are formed with inorganic acids such as, for example, hydrochloric or phosphoric acids, or such organic acids as acetic, oxalic, tartaric, mandelic, and the like. Salts formed from free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

To immunize a subject, the polypeptide of interest, or an immunologically active fragment thereof, is administered parenterally, usually by intramuscular injection in an appropriate vehicle. Other modes of administration, however, such as subcutaneous, intravenous injection and intranasal delivery, are also acceptable. Injectable vaccine formulations will contain an effective amount of the active ingredient in a vehicle, the exact amount being readily determined by one skilled in the art. The active ingredient may typically range from about 1% to about 95% (w/w) of the composition, or even higher or lower if appropriate. The quantity to be administered depends on the animal to be treated, the capacity of the animal's immune system to synthesize antibodies, and the degree of protection desired. Effective dosages can be readily established by one of ordinary skill in the art through routine trials establishing dose response curves. The subject is immunized by administration of the particular antigen or fragment thereof, or analog thereof, in at least one dose, and preferably two doses. Moreover, the animal may be administered as many doses as is required to maintain a state of immunity to pneumonia. An alternative route of administration involves gene therapy or nucleic acid immunization. Thus, nucleotide sequences (and accompanying regulatory elements) encoding the subject proteins can be administered directly to a subject for in vivo translation thereof. Alternatively, gene transfer can be accomplished by transfecting the subject's cells or tissues ex vivo and reintroducing the transformed material into the host. DNA can be directly introduced into the host organism, i.e., by injection.

Targeting agents, such as antibodies directed against surface antigens expressed on specific cell types, can be covalently conjugated to the liposomal surface so that the nucleic acid can be delivered to specific tissues and cells susceptible to *A. pleuropneumoniae*.

The following examples are for the purpose of better understanding the present invention but are in no way to be considered as limiting the invention.

### TABLE LEGENDS

**Table 1.** Influence of culture conditions on adhesion *of A. pleuropneumoniae* (*A. plpn*) serotype 2, 5 and 9 strains to porcine alveolar epithelial cells.

**Table 2.** Effect of preptreatment of the bacterial cells with heat, enzymes and sodium metaperiodate on the adhesion of *A. pleuropneumoniae* strains to alveolar epithelial cells.

### EXAMPLES

### Materials and Methods

### Bacterial strains and culture conditions

Three reference strains representing serotypes 2 (strain 266/7656), 5a (strain 1736) and 9 (strain 13261) were used in this study. Stock suspensions were stored at -70°C in RPMI 1640 medium supplemented with 10% non-essential amino acids (Gibco Europe, Merelbeke, Belgium), 10% glutamine, 10% fetal calf serum (FCS) and 1% sodium pyruvate (Gibco) (leukocyte medium). To study the influence of culture conditions on adhesion and expression of surface antigens (capsule, fimbriae and outer membrane proteins), bacteria were grown on Columbia agar supplemented with 1% horse serum and 0.001% NAD for 20 hours at 37°C in an atmosphere with 5% CO₂ (NAD-restricted medium) or on Columbia agar supplemented with 3% horse serum, 0.03% NAD and 5% yeast-extract for 6 hours at 37°C (NAD-rich medium). To study the effect of pretreatment of the bacteria or alveolar epithelial cells, bacteria were grown on the NAD-restricted medium.

### Adherence assay

Alveolar epithelial cells (AEC) were obtained as previously described (29). For the adherence assay, 10⁵ viable cells in 100 µl of supplemented Modified Eagle Medium (MEM, Gibco), were added to wells of 24-well microtiter plates with coverslips and incubated for 3 hours at 37°C in an atmosphere with 5% CO₂. Then, 1 ml of supplemented MEM was added to each well and the plates were further incubated for 2 days. Subsequently, plates were washed once with phosphate buffered saline solution (PBSS) and cells were fixed with 1 ml of methanol 100%. After washing the cells with aqua dest., adhesion tests were performed.

Therefore, 10⁸ Colony Forming Units (CFU) in 1 ml inoculum of the *A*. *pleuropneumoniae* strains were added to the cultures. The microtiter plates were centrifuged at 110 x g for 10 minutes at 37°C and further incubated at 37°C during 30 minutes in an atmosphere with 5% CO₂. Then plates were washed 4 times with aqua dest., stained with Haemacolor® staining reagents (Merck, Darmstadt, Germany) and microscopically examined.

In each assay, one hundred cells were examined for the presence of adhering bacteria. The following scores were used for each alveolar epithelial cell: score 0 = no adhesion, score 1 = 1 to 20 bacteria adhered to the cell, score 2 = more than 20 bacteria adhered to the cell. The number of alveolar epithelial cells with score 0, 1 or 2 was determined.

### Influence of culture conditions on adhesion to AEC and expression of capsule, fimbriae and outer membrane proteins

### Influence of culture conditions on adhesion

To study the influence of the culture conditions on adhesion, adhesion tests were performed with *A*. *pleuropneumoniae* biotype 1-serotype 2, 5a and 9 strains grown on either the NAD-restricted or NAD-rich medium.

### Influence of culture conditions on expression of capsule

*A. pleuropneumoniae* biotype 1-serotype 2, 5a and 9 strains were grown on the NAD-restricted and the NAD-rich medium. After incubation, bacteria were harvested, washed once with PBSS and resuspended in PBSS at a concentration of 10⁸ CFU/ml. In order to avoid collapse of the capsules during the electron microscopic examination, bacterial suspensions were exposed to undiluted homologous antiserum for 1 hour at 4°C. The antisera were raised in rabbits against whole bacterial cells. The titers of agglutinating antibodies of the rabbit antisera varied between 1:8 and 1:32. Bacterial cells were then suspended in cacodylate buffer (0.1M, pH 7.0) containing 5% glutaraldehyde and 0.15% (w/v) ruthenium red. Fixation was done for 2 hours at 20°C. Thereafter, bacterial cells were immobilized in 3% agar, washed five times in cacodylate buffer with 0.05% ruthenium red and postfixed with 1% osmium tetroxide and 0.05% ruthenium red for 2 hours. Washings were repeated as above, and the samples were dehydrated in a graded series of acetone washes. All the solutions used in processing the specimen, from the wash after glutaraldehyde fixation to dehydration with the 70% acetone solution, contained 0.05% ruthenium red. Samples were then washed twice in propylene oxide and embedded in Spurr low-viscosity resin. Thin sections were cut and stained with uranyl acetate and lead citrate. They were examined with a transmission electron microscope (Philips 201) at an accelerating voltage of 60 kV.

The capsule thickness was measured on micrographs taken during the electron microscopic observations. The cell wall thickness was measured between the inner edge of the capsule layer and the outer leaflet of the plasma membrane.

### Influence of culture conditions on expression of fimbriae

*A. pleuropneumoniae* biotype 1-serotype 2, 5a and 9 strains were grown on the NAD-restricted and the NAD-rich medium, washed once in PBSS and then suspended in a minimal amount of distilled water at a concentration of 10⁸ CFU/ml. The cultures were examined for the presence of fimbriae by negative staining and shadowing. For negative staining, a Formvar-coated copper grid was floated on the surface of the bacterial suspension for 10 minutes. The grid was then floated for 10 seconds on a solution of 2% uranyl acetate in distilled water. After staining, the grid was washed twice and examined with a Philips 201 transmission electron microscope (TEM). For shadowing, the bacterial suspension was absorbed on carbon coated grids and rotary shadowed with platinum-carbon at a small angle (5-6°) with the Balgers shadowing apparatus. The grid was examined with a Philips 201 TEM.

The size of the fimbriae was measured on the micrographs taken during electron microscopic observations.

### Influence of culture conditions on expression of outer membrane proteins

Sarcosyl insoluble outer membrane proteins (OMPs) of *A*. *pleuropneumoniae* biotype 1-serotype 2, 5 and 9 strains grown on the NAD-restricted and the NAD-rich medium were prepared (7). Briefly, 2x10⁹ CFU were suspended in 1 ml of 10 mM HEPES (Gibco, Ghent, Belgium) buffer (pH 7.5). Cells were disrupted by ultrasound with 10 bursts, 20 seconds each, with cooling on ice. Debris was removed by centrifugation at 2000xg for 20 minutes and a 0.5 volume of 2% (w/v) Sarcosyl (Sigma Chemical Co, St Louis, Mo, USA) was added to the supernatant. After incubation for 10 minutes at room temperature, the outer membrane fraction was pelleted by centrifugation at 60,000xg for 1 hour and 40 minutes. The pellet was suspended in 10 mM HEPES buffer and treated with 1 volume of 2% Sarcosyl for 20 minutes at room temperature. The OMP fraction was again pelleted by centrifugation at 60,000xg for 1 hour and 40 minutes and suspended in 1 ml of 10mM HEPES. Before storage at -70°C, the protein concentration was determined (BIORAD Protein Assay, BIORAD, Brussels, Belgium). Then, discontinuous sodium dodecylsulphate polyacrylamide gel electrophoresis (SDS-PAGE) was performed with 5 µg of each sample.

Electrophoresis experiments were performed with a 4.5% stacking gel and a 12% running gel. Each antigen preparation was mixed with equal volumes of solubilization buffer [4% (w/v) of SDS, 10% (v/v) 2-mercapto ethanol, 17.4% (v/v) glycerol and 0.01% bromophenol blue in 0.06M Tris HCl (pH 6.8)] and boiled for 5 minutes prior to electrophoresis. After electrophoresis, silver staining was performed (18).

### N-terminal amino acid sequence analysis of an outer membrane protein

The OMPs of the *A. pleuropneumoniae* biotype 1-serotype 5 strain nr 1736 grown on the NAD-restricted medium were separated by SDS-PAGE, blotted onto a polyvinylidene difluoride membrane and stained with Coomassie blue. A 55 kDa OMP band was cut out and was put in a blotcartridge (cross-flow type). The N-terminal amino acid sequence was analysed in an automatic gas-phase sequencermachine (model 476, Applied Biosystems, Perkin Elmer) using the Edman degradation method. Thereafter, a BLAST (Basis Local Alignment Search Tool) search was performed in the following databases: non-redundant GenBank CDS translations, PDB, SwissProt, Spupdate and PIR.

### Effect of pretreatment of bacterial cells with enzymes, heat, sodium metaperiodate, tetramethylurea and carbohydrates on adhesion to alveolar epithelial cells

*A. pleuropneumoniae* biotype 1-serotype 2, 5a and 9 strains were grown on the NAD-restricted medium and suspended in PBSS at a concentration of 10⁸ CFU/ml. The suspensions were subjected to the following treatments: (1) bacteria were incubated for 30 minutes at 37°C with pepsin solution [1 mg/ml pepsin in 50 mM citrate-10 mM phosphate buffer (pH 3)]; (2) bacteria were incubated for 1 hour at 37°C with trypsin solution [1 mg/ml trypsin in PBSS (pH 7.5)]. Thereafter, the activity of trypsin was stopped by adding soybean trypsin inhibitor (200 U/ml; Sigma) in a sodium phosphate buffer (pH 7.6); (3) bacteria were incubated for 1 hour at 37°C in PBSS (pH 7.3) followed by an incubation for 40 minutes at 37°C in pronase solution [0.01M sodium acetate and 0.005M calciumacetate buffer (pH 7.5) containing 1 mg/ml pronase]; (4) bacteria were incubated for 1 hour at 37°C in a sodium metaperiodate solution [10 mg/ml sodium metaperiodate in PBSS (pH 7.3)] followed by an incubation for 40 minutes at 37°C in the sodium acetate and calciumacetate buffer without pronase; (5) bacteria were incubated for 1 hour at 37°C in the sodium metaperiodate solution followed by an incubation for 40 minutes at 37°C in the pronase solution; (6) bacteria were heated at 56°C for 10, 20 or 30 minutes; (7) bacteria were incubated for 1 hour at 37°C with the following carbohydrates: D-arabinose, D-maltose, D-saccharose, D-mannose, D-glucosamine, D-galactose, N-acetyl-D-galactosamine, D-xylose or D-glucose (all carbohydrates at a final concentration of 0.2M); (8) bacteria were incubated for 1 h at 37°C with 0.01M of the hydrophobic bond-breaking agent 1, 1, 3, 3-tetramethylurea (TMU; Sigma Chemical Co., St. Louis, Mo.).

Controls consisted of non-heat treated bacteria or bacteria incubated in the buffers without pepsin, trypsin, pronase, sodium metaperiodate, carbohydrates or tetramethylureum. After the treatments with pepsin, trypsin, pronase, sodium metaperiodate or carbohydrates, bacterial suspensions were washed in PBSS, resuspended with PBSS at 10⁸ bacteria/ml and used in the adhesion tests. After the treatment with tetramethylureu, adhesion tests were performed in the presence of tetramethylurea.

For each treatment, after incubation, the influence on the viability of the bacterial cells was determined by plating tenfold dilutions on Columbia agar supplemented with 3% horse serum, 0.03% NAD and 5% yeast extract and comparing the number of CFU/ml with controls.

All parts of the experiment were performed in duplicate.

### Effect of pretreatment of the alveolar epithelial cells with proteolytic enzymes, sodium metaperiodate and carbohydrates

After fixation of the alveolar epithelial cells with 100% methanol, cells were washed with aqua dest. and the following treatments were performed: (1) incubation of the cells for 5 minutes at 37°C in the pronase solution containing 1 mg/ml or 0.4 mg/ml of pronase; (2) incubation of the cells for 5 minutes at 37°C in the trypsin solution; (3) incubation of the cells for 1 hour at 37°C in the sodium metaperiodate solution; (4) incubation of the cells for 1 hour at 37°C with either D-mannose, D-maltose, D-xylose, D-arabinose and D-saccharose at a final concentration of 0.2M.

After incubation of the cells with trypsin, pronase or sodium metaperiodate, cells were washed three times with PBSS. After incubation of the cells with the sugars, cells were not washed. Adhesion assays were performed with *A. pleuropneumoniae* biotype 1-serotype 2, 5a and 9 strains grown on the NAD-restricted medium as described above. Controls consisted of cells incubated in the same buffer without enzymes, sodium metaperiodate or carbohydrates. All parts of the experiment were repeated at least 3 times.

### Statistical analysis

The influence of the culture conditions and the different pretreatments of the bacterial cells and the alveolar epithelial cells on adhesion was compared with the Kruskal-Wallis one way non-parametric analysis of variance test (computer program STATISTIX 4.1, Analytical software). A significance level of 0.05 was used.

### RESULTS

### 1. Influence of culture conditions on adhesion and expression of capsule, fimbriae and outer membrane proteins

### Influence of culture conditions on adhesion

The number of alveolar epithelial cells with adhesion scores 0, 1 or 2 is given in Table 1 for *A. pleuropneumoniae* biotype 1-serotype 2, 5a and 9 stains grown on the NAD-rich and the NAD-restricted medium. For the serotype 5a and 9 strains, adhesion scores were significantly higher when bacteria were grown under NAD-restricted conditions. For the serotype 2 strain, there were no significant differences in adhesion scores between the two growth conditions.

Bacteria were not covering the whole surface of the alveolar epithelial cells; they were only attached to localized areas of the alveolar epithelial cells, a pattern which is also known as localized adherence pattern (Figure 1).

### Influence of culture conditions on expression of capsule

Bacterial cells of *A*. *pleuropneumoniae* biotype 1-serotype 2, 5a and 9 strains were covered with a layer of capsular material (Figure 2). The thickest capsular layer (40-100 nm) was seen around cells from the serotype 2 and 5a strains grown in NAD-rich conditions. These capsules had a regular, continuous appearance. The capsule of these strains was thinner and more irregular when grown in NAD-restricted conditions (20 to 50 nm). The serotype 9 strain had an irregular capsule with a thickness between 20 to 80 nm irrespective of the growth conditions.

### Influence of culture conditions on expression of fimbriae

Fimbriae extending outward from the cell walls were demonstrated on all three *A. pleuropneumoniae* strains grown on the NAD-restricted medium and on the A. *pleuropneumoniae* biotype 1-serotype 2 strain grown on the NAD-rich medium. The fimbriae were most easily observed when rotary shadowing was used. They were filamentous, had a length of 400-800 nm and a width of 5-8 nm (Figure 3). The density of the fimbriae varied from cell to cell but was in general low. Not all cells in a culture were fimbriated.

Fimbriae as described above could not be detected on the serotype 5a and 9 strains grown on the NAD-rich medium.

### Influence of culture conditions on expression of outer membrane proteins

SDS-PAGE analysis of the sarcosyl-insoluble OMP fraction of *A. pleuropneumoniae* serotype 2, 5a and 9 strains grown on the NAD-rich or on the NAD-restricted medium are shown in Figure 4. In the OMP fraction of the serotype 5a and 9 strains, a 55 kDa band was observed when bacteria were grown in NAD-restricted conditions. This band was absent or much weaker when bacteria were grown in NAD-rich conditions. The 55 kDa band was also present in the OMP fraction of the serotype 2 strain, but for this serotype, no differences between the two growth conditions were observed.

### 2. N-terminal amino acid sequence analysis of the 55 kDa protein band

For the analysis of the N-terminal amino acid sequence of the 55 kDa OMP of the *A. pleuropneumoniae* biotype 1-serotype 5a strain, twenty-six cycle were run. The following main sequence was found: Gly-Lys-Asp-Leu-Asn-Val-Phe-Asp-Lys-Asn-Tyr-Gly-Leu-Leu-Ile-Asn-Gly-Lys-(Gln)-Thr-Gln/Ala-Phe-Arg-Ser-Gly/Asp-Asp (SEQ ID NO 1).

No homology with other protein sequences was found in the databases.

### 3. Effect of pretreatment of bacterial cells with enzymes, heat, sodium metaperiodate, tetramethylurea and carbohydrates on adhesion to alveolar epithelial cells

Results are presented in Table 2. Treatment of the bacterial cells with enzymes, heat and sodium metaperiodate significantly decreased the adhesion of the serotype 2 strain. Treatments could be arranged in descending degree of inhibition as follows: combination of sodium metaperiodate and pronase, heat, pronase, pepsin, trypsine and sodium metaperiodate. For the serotype 5a strain, single sodium metaperiodate treatment had no significant effect on the adhesion. Treatment of the bacterial cells with enzymes and heat significantly decreased the adhesion. The highest inhibition was found with heat and a combination of sodium metaperiodate and pronase followed by pronase, pepsin and trypsin. For the serotype 9 strain, all treatments significantly inhibited the adherence. In descending order, inhibition was observed with heat, combination of sodium metaperiodate and pronase, pronase, pepsin, trypsine and sodium metaperiodate.

Tetramethylurea or carbohydrate treatment did not reduce the level of adhesion.

Pronase, trypsin, TMU and carbohydrate treatment of the bacterial cells did not have any effect on the viability. Treatment of the bacteria with pepsin and sodium metaperiodate resulted in a decrease in viability.

### 4. Effect of pretreatment of the alveolar epithelial cells with enzymes, sodium metaperiodate and carbohydrates

Treatment of the cells with 1 mg/ml of pronase could not be interpreted since the alveolar epithelial cells were destroyed. Treatment of the cells with 0.4 mg/ml did not result in destruction of the alveolar epithelial cells and did not affect the adhesion of the bacteria. Trypsin, pepsin, sodium metaperiodate or carbohydrate treatment of the alveolar epithelial cells had no effect on the adhesion of the bacteria.

### REFERENCES

1. **Belanger M., Dubreuil D., Harel J., Girard C. and Jacques M..** 1990. Role of lipopolysaccharides in adherence of *Actinobacillus pleuropneumoniae* to porcine tracheal rings. Infect. Immun. **58**:3523-3530.

2. **Belanger M., Dubreuil D. and Jacques M..** 1994. Proteins found within porcine respiratory tract secretions bind lipopolysaccharides of *Actinobacillus pleuropneumoniae.* Infect. Immun. **62**:868-873.

3. **Belanger, M., Rioux S., Foiry B. and Jacques M..** 1992. Affinity for porcine respiratory tract mucus is found in some isolates of *Actinobacillus pleuropneumoniae.* FEMS Microbiol.Lett. **76**:119-125.

4. **Chiers K., Haesebrouck F., Van Overbeke I., Charlier G. and Ducatelle R.** 1999. Early in vivo interactions of *Actinobacillus pleuropneumoniae* with tonsils of pigs. Vet. Microbiol. **68**:301-306.

5. **Confer A., Clinckenbeard K and Murphy G.** 1995. Pathogenesis and virulence of *Pasteurella haemolytica* in cattle: an analysis of current knowledge and future approaches. *Haemophilus*, *Actinobacillus* and *Pasteurella.* Donachie W., Lainson F. and Hodgson J., Plenum Press, New York, 51-62.

6. **Dekker N., Lammel C., Mandrell R. and Brooks G.** 1990. Opa (protein II) influences gonococcal organization in colonies, surface appearance, size and attachment to human fallopian tube tissues. Microb. Pathog. **9**:19-31.

7. **Deneer H. and Potter A.** 1989. Identification of a maltose-induced major outer membrane protein in *Actinobacillus (Haemophilus) pleuropneumoniae*. Microb. Pathog. **6**:425-432.

8. **Doig P., Sastry P., Lee K., Hodges R., Paranchych W and Irvin R.** 1988. Role of pili in adhesion of *Pseudomonas aeruginosa* to human respiratory epithelial cells. Infect. Immun. **56**:1641.

9. **Dom P., Haesebrouck F., Ducatelle R. and Charlier G.** 1994. In vivo association of *Actinobacillus pleuropneumoniae* with the respiratory epithelium of pigs. *Haemophilus*, *Actinobacillus* and *Pasteurella.* Donachie W., Lainson F.A. and Hodgson J.C., Plenum Press, New York and London, 204.

10. **Dom P., Haesebrouck F., Ducatelle R. and Charlier G..** 1994. In vivo association of *Actinobacillus pleuropneumoniae* serotype 2 with the respiratory epithelium of pigs. Infect. Immun. **62**:1262-1267.

**11. Enriquez I., Guerrero A., Serrano J., Rosales M., Hamer R., Martinez R., Godinez D. and de la Garza M.** 1999. Adhesion of *Actinobacillus pleuropneumoniae* to type III swine lung collagen. *Haemophilus*, *Actinobacillus* and *Pasteurella* conference, P3.

12. Haesebrouck F., Chiers K., Van Overbeke I. and Ducatelle R.. 1997. *Actinobacillus pleuropneumoniae* infections in pigs: the role of virulence factors in pathogenesis and protection. Vet. Microbiol. 58:239-249.

13. **Hamer R., Enriquez I., Godinez D., Guerrero A., Martinez R., Talamas P., Vaca S., Garcia C. and de la Garza M.** 1999. Adhesion of *Actinobacillus pleuropneumoniae* to swine buccal epithelial cells. *Haemophilus*, *Actinobacillus* and *Pasteurella* conference, P2.

14. **Jacques M.** 1999. Adherence of mini-Tn*10*-generated, surface polysaccharides mutants of *Actinobacillus pleuropneumoniae* serotype 1. *Haemophilus*, *Actinobacillus* and *Pasteurella* conference, A3.2.

15. **Jacques M., Belanger M., Roy G. and Foiry B.**. 1991. Adherence of *Actinobacillus pleuropneumoniae* to porcine tracheal epithelial cells and frozen lung sections. Vet. Microbiol. **27**:133-143.

16. **Jacques M., Roy G. and Mittal K..** 1988. Hemagglutinating properties of *Actinobacillus pleuropneumoniae*. Can. J. Microbiol. **34**:1046-1049.

17. **Jolie R., Mulks M. and Thacker B..** 1994. Antigenic differences within *Actinobacillus pleuropneumoniae* serotype 1. Vet. Microbiol. **38:**329-349.

18. **Morrissey J.** 1981. Silver staining for proteins in polyacrylamide gels: a modified procedure with enhanced uniform sensitivity. Anal. Biochem. **117**:307-310.

19. **Nielsen R.** 1986. Serological characterization of *Actinobacillus pleuropneumoniae* strains and proposal of a new serotype: serotype 12. Acta Veterinaria Scandinavica **27**:453-455.

20. **Ofek I. and Beachy E.** 1980. Bacterial adherence. Adv. Int. Med. **25:**505-532.

21. **Ofek I. and Doyle R.** 1994. Bacterial adhesion to cells and tissues.

22. **Paranchych W. and Frost L.** 1988. The physiology and biochemistry of pili. Adv. Microb. Physiol. **29**:53.

23. **Ramphal R., Koo L., Ishimoto K., Totten P., Lara J. and Lory S.** 1991. Adhesion of *Pseudomonas aeruginosa* pilin-deficient mutants to mucin. Infect. Immun. **59**:1307-1311.

**24. Read R., Rutman A., Jeffrey P., Lund V., Brain A., Moxon R., Cole P. and Wilson R.** 1992. Interaction of capsulated *Haemopilus influenzae* with human airway mucosa in vitro. Infect. Immun. **60:**3244-3252.

25. **Sato H. and Okinaga K.** 1987. Role of pili in the adherence of *Pseudomonas aeruginosa* to mouse epidermal cells. Infect. Immun. **55:**1774.

26. **Scaletsky I., Silva M. and Trabulsi L.** 1984. Distinctive patterns of adherence of enteropathogenic *Escherichia coli* to HeLa cells. Infect. Immun. **45:**534-536.

27. **Scotland S., Richmond J. and Rowe B.** 1983. Adhesion of enteropathogenic strains of *Escherichia coli* (EPEC) to HEp-2 cells is not dependent on the presence of fimbriae. FEMS Microbiol.Lett. **20:**191-195.

28. **Starr M., Adler B., Ruffolo C., Al-Hasani K. and Robins-Browne R.** 1999. *htf*A, A novel gene mediating adhesion of nontypeable *Haemophilus influenzae* to HEP-2 cells. *Haemophilus*, *Actinobacillus* and *Pasteurella* conference, A3.9.

**29. Van de Kerkhof A., Haesebrouck F., Chiers K., Ducatelle R., Kamp E. and Smits M..** 1996. Influence of *Actinobacillus pleuropneumoniae* and its metabolites on porcine alveolar epithelial cells. Infect. Immun. **64:**3905-3907.

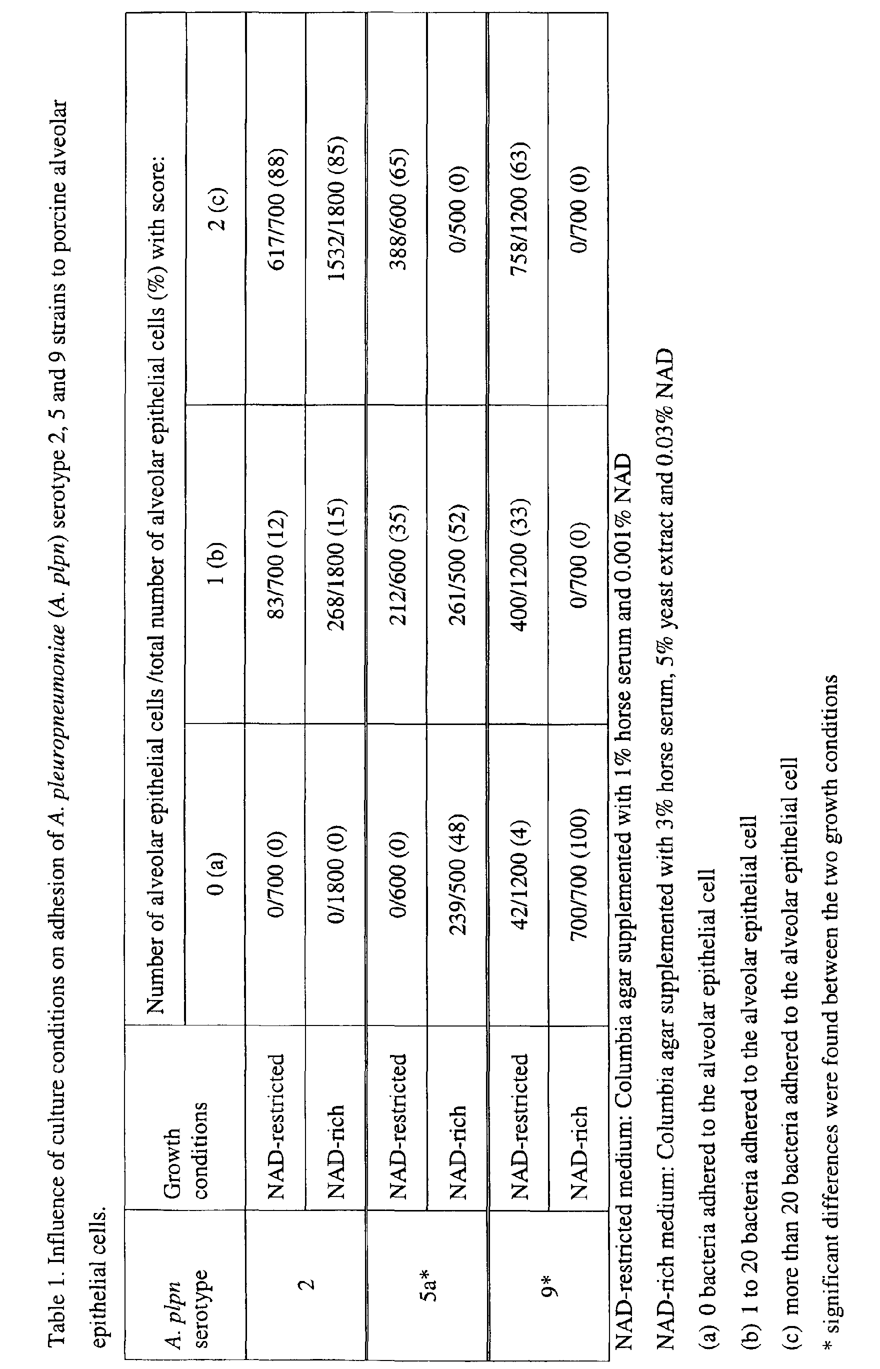

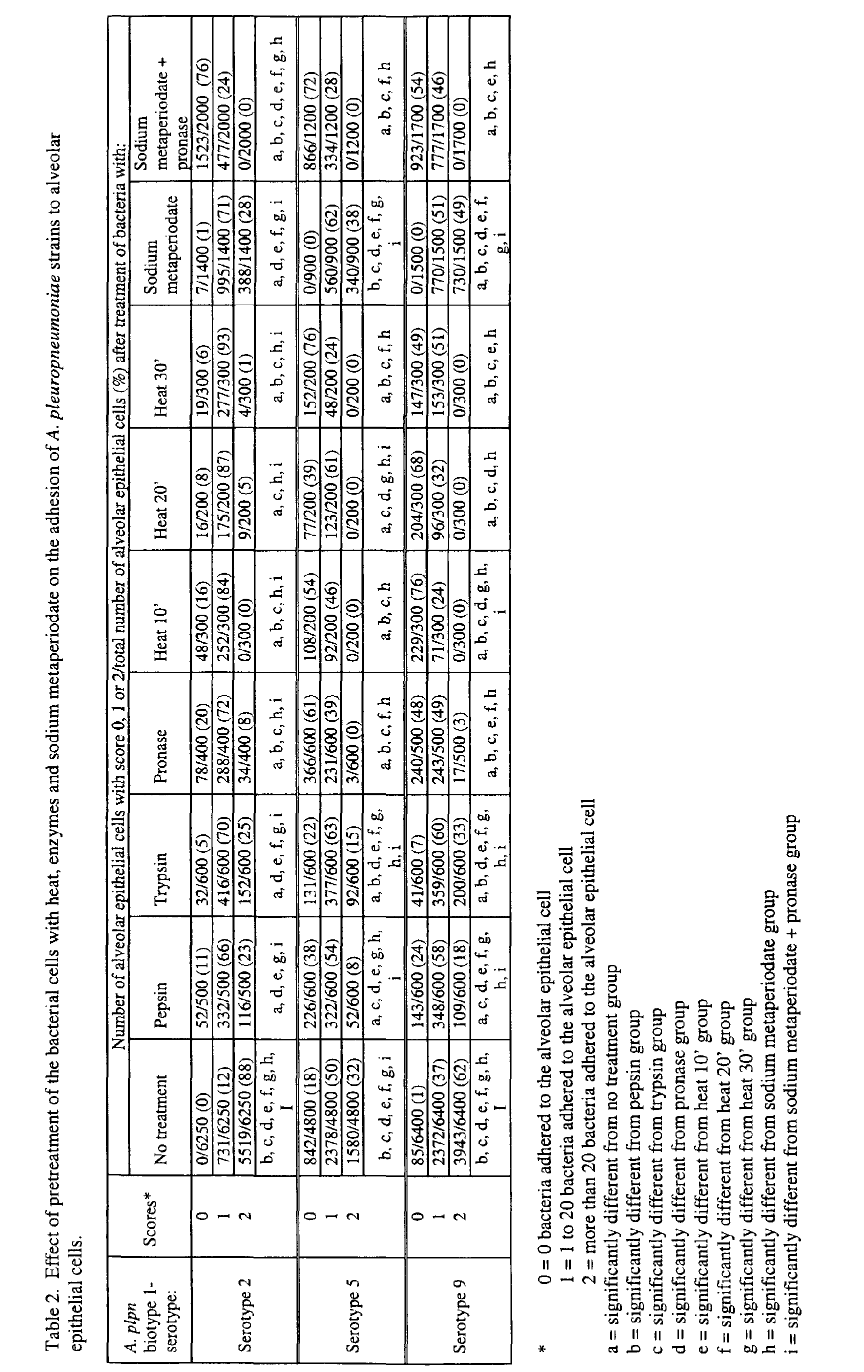

## Claims

1. Purifed immunogenic *Actinobacillus pleuropneumoniae* outer membrane protein having an N-terminal amino acid sequence as shown in SEQ ID NO 1 and having a molecular weight of about 55 kD or an immunogenic fragment thereof.

2. A polynucleic acid comprising a sequence of at least 10 contiguous nucleotides selected from:
(a) a polynucleic acid sequence which codes for a polypeptide according to claim 1,
(b) a polynucleic acid sequence which is degenerate as a result of the genetic code to the polynucleic acid sequence as defined in (a), and which still encodes a polypeptide according to claim 1, or
(c) a polynucleic acid sequence which hybridizes to any of the polynucleic acids as defined in (a) or (b).

3. An oligonucleotide probe comprising a fragment of a polynucleic acid according to claim 2, with said probe being able to act as a specific hybridization probe for detecting the presence of *Actinobacillus pleuropneumoniae* polynucleic acids in a sample.

4. An oligonucleotide primer comprising a fragment of a polynucleic acid according to claim 2, with said primer being able to initiate specific amplification of *Actinobacillus pleuropneumoniae* polynucleic acids in a sample.

5. An antibody, more particularly a monoclonal antibody, characterized in that it specifically recognizes an *Actinobacillus pleuropneumoniae* polypeptide according to claim 1, or an immunogenic fragment thereof.

6. A method for detection of *Actinobacillus pleuropneumoniae* comprising the steps of contacting a sample taken from said individual with a polypeptide or fragment thereof according to claim 1 under conditions allowing the formation of an immunological complex, and subsequently detecting the complexes formed.

7. A method for detection of *Actinobacillus pleuropneumoniae* comprising the steps of contacting a sample taken from an individual with an oligonucleotide probe according to claim 3, under conditions allowing the formation of a hybridization complex, with said polynucleic acids of said sample being possibly amplified prior to hybridization, and subsequently detecting the complexes formed.

8. A method for detection of *Actinobacillus pleuropneumoniae* comprising the steps of contacting a sample taken from an individual with an oligonucleotide primer according to claim 4, under conditions allowing the specific amplification of polynucleic acids, and sequencing the amplified polynucleic acids or detecting the amplified polynucleic acids with suitable nucleotide probes.

9. A method for diagnosis of *Actinobacillus pleuropneumoniae* infection in an individual comprising the steps of contacting a sample taken from said individual, with an antibody according to claim 5, under conditions allowing the formation of an immunological complex, and subsequently detecting the complexes formed.

10. A method for detecting and/or quantifying the cellular immune response of an individual against a polypeptide as defined in claim 1, said method comprising:
- measuring a delayed type hypersensitivity reaction upon subcutaneous injection of said polypeptide in said individual, or
- measuring the stimulation of periferal blood lymphocytes isolated from the individual to be tested, upon addition of said polypeptide to said lymphocytes under conditions allowing the immune recognition of said polypeptide.

11. A method for detecting individuals having been in contact with *Actinobacillus pleuropneumoniae* comprising:
- contacting a polypeptide according to claim 1 with the cellular immune system of said individual, either in vitro or in vivo, and,
- detecting and/or quantifying the cellular immune response raised against said polypeptides by means of a method according to claim 10.

12. A recombinant *Actinobacillus pleuropneumoniae* strain in which the gene(s) encoding at least one of the polypeptides according to claim 1 has (have) been deleted or inactivated.

13. A vaccine composition comprising as an active component a polypeptide according to claim 1 or a fragment thereof, or a polynucleic acid according to claim 2, or a recombinant *Actinobacillus pleuropneumoniae* strain according to claim 12, said active component being combined with a pharmaceutically acceptable carrier.

14. A combined method for vaccination against and detection of pleuropneumonia, said method comprising:
- vaccinating an individual liable to become infected with pleuropneumonia with a recombinant *Actinobacillus pleuropneumoniae* strain according to claim 12, and,
- detecting in an individual liable to be infected with pleuropneumoniae an immune response against a polypeptide according to claim 1, by means of a method according to any of claims 6, 9 or 10, wherein said combined method is characterized in that it enables the differentiation between vaccinated and field-infected individuals.

15. A DNA construct comprising a polynucleic acid according to claim 2, or a part thereof and wherein the coding sequence of said polynucleic acid is operably linked to a control sequence enabling the expression of the coding sequence by a specific host.

16. A host cell transformed with a DNA construct according to claim 15.

17. A recombinant polypeptide encoded by a polynucleic acid according to claim 2 or part thereof, said recombinant polypeptide being produced by:
- transforming a DNA construct according to claim 15 into a suitable host cell,
- culturing said transformed cellular host under conditions, which allow the expression and possibly secretion of the encoded polypeptide, and
- recovering the expressed polypeptide from the culture.

18. A kit for the detection of *Actinobacillus pleuropneumoniae* comprising a polypeptide or peptide according to claim 1, or a recombinant peptide according to claim 17, preferentially in combination with other polypeptides or peptides from *Actinobacillus pleuropneumoniae*, with said (poly)peptides being preferably immobilized on a solid substrate.

19. A kit for the detection of *Actinobacillus pleuropneumoniae* comprising an antibody according to claim 5, with said antibody being preferably bound to a solid support.

20. A kit for the detection of *Actinobacillus pleuropneumoniae* comprising:
- a primer according to claim 4, and/or
- an oligonucleotide probe as according to claim 3, with said probe being preferentially immobilized on a solid substrate.

21. A kit for combined vaccination against and detection of pleuropneumonia, with said kit comprising at least the following components:
- a vaccine composition comprising as an active principle a recombinant *Actinobacillus pleuropneumoniae* strain according to claim 12, and,
- a polypeptide or peptide according to any of claims 1 and 2, or a recombinant polypeptide according to claim 17,
said kit being further characterized by the fact that its components enable the differentiation between vaccinated and field-infected individuals.

22. The combined use of:
- a recombinant *Actinobacillus pleuropneumoniae* vaccine strain according to claim 12 for use in vaccination, and,
- a polypeptide or peptide according to claim 1 or a recombinant polypeptide according to claim 17 for use in a diagnostic method.

23. A polypeptide or fragment thereof according to claim 1 or a recombinant protein according to claim 17, or an antibody according to claim 5, or a polynucleic acid according to any of claims 2, 3 or 4, for use as a medicament.

24. A polypeptide or a fragment thereof according to claim 1 or a recombinant polypeptide according to claim 17, or an antibody according to claim 5, or a polynucleic acid according to any of claims2, 3 or 4, for the manufacture of a medicament, more particularly for the preparation of a vaccine or for the preparation of a diagnostic composition.

25. A method for producing an immunogenic *Actinobacillus pleuropneumoniae* outer membrane protein according to claim 1 or 17 comprising growing a host cell according to claim 16 under conditions whereby the protein encoded by said DNA construct is expressed, thereby producing said outer membrane protein according to claim 1 or 17.
